# EUROPEAN PATENT SPECIFICATION

(11) **EP 1 392 912 B1**
(45) Date of publication and mention of the grant of the patent: **16.11.2005**
(21) Application number: 01935249.1
(22) Date of filing: 10.05.2001
(51) Int. Cl.: D06M 23/12, D06M 15/643, D06M 15/263, D06M 15/267, D06P 1/44

(54) **PROCESS FOR APPLYING MICROCAPSULES TO TEXTILE MATERIALS**
VERFAHREN ZUM AUFBRINGEN VON MIKROKAPSELN AUF TEXTILGÜTER
PROCEDE PERMETTANT D'APPLIQUER DES MICROCAPSULES SUR DES MATIERES TEXTILES

(30) Priority: 09.05.2001 US 852313
(43) Date of publication of application: 03.03.2004
(73) Proprietor: Sara Lee Corporation, Winston-Salem, NC 27105 (US)
(72) Inventor: HARRIS, Larry, King, NC 27021 (US); BLEVINS, Larry, Matthews, NC 28104 (US)
(74) Representative: Boff, James Charles
(86) International application number: PCT/US2001/015067
(87) International publication number: WO 2002/090643

(56) References cited:
- EP-A- 0 436 729
- EP-A- 0 581 274
- US-A- 5 298 035

## Description

### FIELD OF THE INVENTION

The present invention relates to a process for applying microcapsules to textile materials. Embodiments of the present invention are advantageous for use in hosiery finishing processes.

### BACKGROUND OF THE INVENTION

A technique known as microencapsulation is used to enclose chemically reactive material in tiny microcapsules from which such material can be released when exposed to particular conditions. See, Fairchild's Dictionary of Textiles, 7^{th} edition. Microcapsules comprise a core including one or more active agents, or ingredients, surrounded by a thin wall. A variety of processes exist for manufacturing microcapsules having varying sizes, alternative materials for the composition, thickness, and rupturability of the capsule wall, and various active agents within the capsule core. Microcapsule walls can comprise a wide variety of materials, such as gelatin and polymeric materials, including polyurethane, polyolefin, polyamide, polyester, polysaccharide, silicone resins, epoxy resins, formaldehyde resins, and the like. Contents are released when microcapsule walls rupture, dissolve, or otherwise disintegrate, in response to appropriate stimuli, or activating factor(s), for example, pressure or friction from physical contact with skin, temperature of skin, and/or wetting by skin moisture.

Microcapsules applied to textile materials, and to fabric and garments made therefrom, impart characteristics supplied by active agents to the textile materials and/or deliver the agents to the fabric or garment environment, such as the wearer of a garment. Active agents may be "conditioning" agents, that is, substances which improve or modify the chemical or physical characteristics of a surface, for example, fabric and/or skin. Examples of fabric conditioning agents include fragrances, softening agents, elasticity improving agents, flame proofing agents, bacteriostatic agents, antistatic agents, soil proofing agents, water repellent agents, antishrinking agents, heat proofing agents, coloring materials, and brighteners, among others. Skin conditioning agents, include, for example, fragrances, moisturizing agents, vitamins, depilatory agents, coloring agents, bleaching agents, and combinations of these and other agents.

A typical approach for applying microcapsules to textile materials is to place a textile material in a bath containing both microcapsules and a binder as an initial step in the application process. For example, U.S. Patent No. 4,882,220 discloses a process that involves applying to a fibrous structure a treating liquid comprising microcapsules enclosing a fragrance, a resinous binder, such as a silicone-based binder in an amount of 0.5-5 times the weight of the microcapsules, and a pressure absorbing agent, and then drying the fibrous structure at a temperature of between 60° and 150° C. As another example, U.S. Patent No. 5,232,769 discloses mixing skin-conditioning microcapsules with a silicone or urethane based resin, feeding the mixture into a bath containing a softening agent, introducing a textile material into the bath, and heating in the range of 20 to 80° C for about 15 to 30 minutes.

Another conventional method for applying microcapsules to textile materials is to expose a textile material to microcapsules coated with a binder. In U.S. Patent No. 4,234,627, for example, microcapsules containing a fabric conditioning agent are applied to fabrics via a capsule transfer agent surrounding, or enveloping, the microcapsules. The capsule transfer agents may be cationic or nonionic organic materials and mixtures thereof having a melting point in the range of 40° to 150° C, which deposit microcapsules onto fabrics during the washing, rinsing, or drying cycles of a laundry process.

In each of these methods, microcapsules are distributed around a textile material in the presence of a binder. Binders cross-link with and cause microcapsules to attach, or bind, to the first surface with which a microcapsule-binder complex comes into contact. As such, microcapsules coated with binders and microcapsules introduced to textile materials in the presence of a binder tend to adhere to the first surface contacted. This tendency results in microcapsules aggregating predominantly on one surface of a textile material, for example, the outer surface of a hosiery garment, creating a layer, or film, of microcapsules on one surface. Consequently, each of these methods has the disadvantage of uneven penetration and distribution of microcapsules around all surfaces of the textile material. Layering of microcapsules on one surface of a textile material resulting from initially dispersing microcapsules in the presence of a binder creates the need for using an increased amount of microcapsules in attempt to apply microcapsules to other surfaces of the textile material. As such, prior textile treatment methods have generally required utilization oflarge numbers of microcapsules to provide effective microcapsule application.

Since microcapsules added to a treatment environment in the presence of a binder tend to adhere to the first surface contacted, microcapsule-binder complexes also aggregate on surfaces in the treatment environment other than a textile material, for example, the wall of a treatment bath container. Thus, methods involving initial application of microcapsules in the presence of a binder also have the disadvantages of decreased distribution of microcapsules around a textile material and loss of microcapsules and binder due to adherence onto other surfaces in a treatment environment. Such uneven distribution and loss of microcapsules and binder results in a need for an increased amount of both microcapsules and binder to adequately cover a textile material.

Uneven distribution of microcapsules throughout a textile material causes an uneven delivery of microcapsule contents, for example, to the wearer of a microcapsule-treated hosiery garment. Moreover, "layered" microcapsules tend to shed, or "flake" away, from the surface of a textile material, causing an undesirable appearance of a fabric or garment.

Other methods attempting to improve penetration and distribution when applying microcapsules to textile materials involve contacting a textile material with a binder before adding microcapsules. One method utilizes a step wherein a textile material is pre-treated with a relatively small amount of binder, after which microcapsules are added, and then a relatively large amount of binder is applied. In another method, disclosed in U.S. Patent No. 4,201,822, microcapsules incorporated in an acrylic resin emulsion are vacuum-filtered onto a fabric, which is allowed to cure at ambient temperature overnight. In yet another method using binder-coated microcapsules, the binding agent is filtered away from the microcapsules so as to allow the binder to transfer to a textile surface first. Filtered microcapsules are then added, and cross-link with the binder on the textile surface.

One disadvantage to approaches applying a binder before microcapsules is similar to that described above. Since a binder adheres to the first surface it contacts, the binder tends to aggregate on one surface of a textile material, and microcapsules will adhere primarily to the surface containing the binder when added. Thus, such techniques do not solve the problem of uneven penetration and distribution when applying microcapsules to textile materials.

Prior methods have also attempted to apply microcapsules without a binder. U.S. Patent No. 4,882,220, discussed above, discloses that for firm retention of microcapsules encapsulating a perfume on ultra fine fibers, for example, 0.7 denier or less/filament, copolymer filaments used in crimping yarns, the microcapsules are trapped between fibers without using binders. However, washing easily removes microcapsules attached to fibers without a binder. Thus, the 4,882,220 patent describes applying microcapsulesto ultra fine fibers in a solution with a binder, for example, a silicone resin binder in the amount of 0.1-2% by weight of the solution, to decrease removal of microcapsules during washing.

These types of approaches to microcapsule application have other disadvantages. When a textile material is contacted with a binder before microcapsules are added, a substantial increase in the amount of binder is required to adhere the microcapsules to the material. An increase in the amount of binder used increases cost. Moreover, when relatively large amounts of binder are used, the tendency of "layered" microcapsules to shed from the surface of a textile material increases, resulting in an undesirable appearance of a fabric or garment. Another disadvantage of such approaches is a longer cycle time to distribute an increased amount of binder around a textile material and to apply binder at multiple stages. A longer cycle time increases cost of the overall process.

Binding agents generally decrease the "hand," or tactile qualities such as softness or "feel," of a fabric or garment. The more binder used in a microcapsule application process, the more likely the hand of a textile material will be diminished. As well, in order to enhance resistance to removal of microcapsules during washing, an increased amount of binder is often used, which impairs the feel of the textile structure even further. In addition, when binders permeate into woven or knitted yarns, an even stiffer hand results. Thus, a disadvantage in such methods applying binder before and/or after microcapsules and using an increased amount of binder is that the hand of the textile material is decreased.

Efforts have been undertaken to address the negative effect on hand caused by use of an increased amount of binder. For example, fabric softening agents are added to the binder in some processes, and a silk protein is added to microcapsules in another process, to counter the decrease in hand associated with use of larger amounts of binder. Other methods pre-treat a textile material with a water repellent to prevent permeation of the adhesive binder into the textile fibers or interstices of fabric knits or weaves in attempt to decrease the adverse effect of binders on the hand of the fabric. However, adding such steps and materials to microcapsule application processes disadvantageously adds to both the cycle time and cost of those processes and may even prevent microcapsules from penetrating into the structure of a textile material.

Thus, there is a need for a process for applying microcapsules to textile materials such as fabric and/or garments that provides for more thorough and more even penetration of microcapsules in a textile material, that avoids aggregation and layering of microcapsules predominantly on one surface, and that decreases and/or overcomes the tendency of layered microcapsules to shed from the surface of a textile material. There is a need for a more efficient process for applying microcapsules to textile materials that overcomes the disadvantages of using increased amounts of microcapsules and binder and increased process cycle times of prior methods. There is also a need for a process for applying microcapsules to fabric and/or garments that provides for an improved hand of the fabric and/or garments. It is to these perceived needs that the present invention is directed.

### SUMMARY OF THE INVENTION

The present invention relates to a process for applying microcapsules to textile materials. The present invention provides processes for treating a textile material with microcapsules, such as in a hosiery finishing process, in which microcapsules are applied to the textile material first, followed by dispersion of the microcapsules with a dispersant, which is followed by addition of a binder to promote adherence of the microcapsules to the textile material.

Textile materials obtained by the process of the present invention have an even distribution of microcapsules and an improved hand when compared to other microcapsule-treated textile materials. Textile materials obtained by the process of the present invention include natural and synthetic textile fibers and yarns, woven and knitted fabrics, non-woven structures, and garments made from such fibers, yarns, and/or fabrics. Processes according to the present invention are particularly well suited for applying microcapsules to textile materials such as fine denier hosiery, including hosiery comprising nylon.

Features of a process of the present invention for applying microcapsules to a textile material may be accomplished in one or more of the embodiments of the present invention. As will be appreciated by those of ordinary skill in the art, the present invention has wide utility in a number of applications as illustrated by the variety of features and advantages discussed below.

A process for applying microcapsules to a textile material of the present invention provides numerous advantages over prior methods. For example, the present invention provides a process for applying microcapsules to a textile material utilizing a sequence of first applying microcapsules without a binder, followed by dispersing the microcapsules through the textile material with a dispersant, and thereafter promoting adherence of the microcapsules to the textile material using a binder. As such, a process of the present invention avoids the tendency associated with previously used methods of microcapsules introduced in the presence of a binder to adhere only to the first surface of a textile material contacted. Thus, a process for applying microcapsules as in the present invention advantageously provides for more thorough and more even penetration of microcapsules in a textile material.

Another advantage of the present invention is that, as a result of more even penetration of microcapsules in a textile material, the microcapsules are less likely to "flake" away from the textile material. This allows a fabric and/or garment made by a process of the present invention to maintain a desirable appearance without flaking. Moreover, a fabric and/or garment made by a process of the present invention has the advantage of maintaining more microcapsules in adherence to the fabric and/or garment for increased delivery of the microcapsule contents over a longer period of time. As a result of such increased distribution and adherence, a process of the present invention advantageously utilizes a much lower percentage of microcapsules relative to a textile material than in prior methods.

Another advantage is that the present invention provides a process for applying microcapsules to textile materials that utilizes a shorter cycle time and is thus less expensive than prior methods. Another advantage of a process for applying microcapsules according to the present invention is that, because a binder is applied after microcapsules are dispersed around and through a textile material, less binder is used, and the process is thus les expensive than prior methods.

Another advantage is that the present invention provides a process for applying microcapsules to textile materials that eliminates the need for additional steps, such as filtration of binder-coated microcapsules, pre-treatment of fabric with a binder, and adding other agents, for example, a protein for softening, as used in prior methods. A process for applying microcapsules as in the present invention requires no complicated processing steps. Consequently, the present invention provides a process for applying microcapsules to textile materials that is more efficient and cost-effective.

Another advantage is that in a process for applying microcapsules using a lesser amount of binder as in the present invention, the hand of the textile material is enhanced, as compared to other methods. Yet another advantage is that in a process for applying microcapsules in which excess binder is rinsed from a textile material as in the present invention, the hand of the fabric and/or garment is enhanced. As such, in a process as in the present invention, microcapsules can be applied to textile structures without impairing the hand, or feel, of the textile structures.

As will be realized by those of skill in the art, many different embodiments of a process for applying microcapsules to textile materials according to the present invention are possible. Additional uses, objects, advantages, and novel features of the invention are set forth in the detailed description that follows and will become more apparent to those skilled in the art upon examination of the following or by practice of the invention.

### DETAILED DESCRIPTION

The present invention relates to a process for applying microcapsules to a textile material. An embodiment of the present invention provides a process for treating textile materials with microcapsules, such as in a hosiery finishing process, in which microcapsules are applied to the textile materials first, followed by dispersion of the microcapsules with a dispersant, which is followed by addition of a binder to promote adherence of the microcapsules to the textile materials.

Textile materials, or structures, treated with microcapsules by processes as in the present invention gradually release encapsulated substances onto a wearer's skin. Such textile structures include apparel which are worn in direct contact with a wearer's skin, such as hosiery of various lengths, including pantyhose, stockings, and socks, underwear, lingerie, foundation garments, leotards, T-shirts, athletic apparel, and the like. Textile structures having microcapsules applied according to processes of the present invention can also be used as bedclothes which can contact the skin, such as mattress covers and sheets, and materials having therapeutic uses, for example gauze, bandages, tape, and the like.

A process for applying microcapsules as in the present invention can be used with textile structures including yarns, fabrics constructed in various manners, such as weaving and knitting in a variety of patterns, and articles and garments made therefrom. Such textile structures may comprise natural fibers such as cotton, synthetic fibers such as nylon and polyester, or mixtures thereof. In a preferred process and product of the present invention, the textile structures to which microcapsules are applied comprise nylon fibers.

In an embodiment of the present invention, a process for applying microcapsules to a textile material comprises contacting the textile material with the microcapsules, dispersing the microcapsules around and through the textile material with a dispersant, and adhering the dispersed microcapsules to the textile material with a binder. In such a process of the present invention, the microcapsules are thoroughly dispersed and evenly applied to the textile material.

In embodiments of the present invention, a textile material may be contacted by microcapsules in a treatment bath container. The textile material is placed in the container. A predetermined weight of microcapsules is measured and diluted with warm water in approximately a 10 to 1 ratio. Microcapsules utilized in processes of the present invention may be of various sizes, have walls of various compositions and thicknesses, and contain various active ingredients. Examples of active ingredients include fragrances, vitamins, and lotions, for example, moisturizing agents such as aloe vera. The microcapsules can contain one or more such active ingredients and combinations of these and/or other active ingredients. The walls of microcapsules of the present invention comprise a gelatin-like substance, and are rupturable in the presence of appropriate stimuli, such as friction and/or body heat.

In embodiments of the present invention, the microcapsule dilution is added to the treatment bath container with the textile material. Preferably, the treatment bath contains no dispersants or binders when the microcapsules are added. In embodiments, microcapsules placed in a bath with the fabric or garment have an anionic charge. The bath is mechanically stirred for three minutes to disperse the microcapsules in the solution around the textile material. This physical dispersion of microcapsules is unaided by other agents, for example, a dispersant or binder.

Once the bath is stirred sufficiently, a dispersant, such as a silicone finish, is placed in the bath to disperse the microcapsules throughout the bath and to thoroughly penetrate the textile material. Dispersants utilized in processes of the present invention have an ionic charge that is different than the charge of the microcapsules. For example, dispersants having a cationic charge are used to disperse microcapsules having an anionic charge. A different ionic charge allows dispersants to disperse the microcapsules in a treatment bath around and through a textile material. A weak, but opposite, charge is often sufficient to provide adequate dispersion of microcapsules. Dispersion of anionic microcapsules around and through a textile material with a mildly cationic dispersant allows the microcapsules to temporarily attach to surfaces of the textile material. In embodiments of the present invention, the dispersant is a silicone finish, such as "SI-1974" manufactured by The Virkler Company (12345 Steele Creek Road, Charlotte, N.C.).

In embodiments, a cationic dispersant is placed in a bath with the textile material. The bath is then heated to a temperature in the range of about 26.67°C to 48.89°C (80° F to 120° F) for a period of between about 8 and 20 minutes. In preferred embodiments, the bath is heated to a temperature of 37.78°C (100° F) for approximately 8 minutes.

Once the bath has been heated for the predetermined temperature and time, a binder is added to the mixture in the bath. As with dispersants described above, binders utilized in processes of the present invention have an ionic charge that is different than the charge of the microcapsules. Binders having a cationic charge are effective for attaching microcapsules having an anionic charge to textile materials. In embodiments of the present invention, anionic microcapsules dispersed around and through, and temporarily attached to, a textile material in a treatment bath with a mildly cationic dispersant are more strongly bound to surfaces of the textile material with a more strongly cationic binder. In embodiments of the present invention, microcapsules are bound to textile materials using a cationic acrylic binder.

After a binder is added, the bath is heated to a temperature in the range of about 26.67°C to 48.89°C (80° F to 120° F) for a period of between about 8 and 20 minutes. In preferred embodiments, the bath is heated to a temperature of 37.78°C (100° F) for approximately 10 minutes. Following heating at the predetermined temperature and time, the treatment container is then drained of the microcapsule and dispersant bath solution.

The textile material is then rinsed to remove excess binder to prevent any excess binder from reacting with a finishing agent. In embodiments, the textile material is rinsed with water having a temperature in the range of about 21.11°C to 43.33°C (70° F to 110° F) for a period of between about 5 and 10 minutes. In preferred embodiments, the textile material is rinsed with circulating water having a temperature of 26.67°C (80° F) for approximately 5 minutes. A warm rinse is preferred in order to effectively remove excess binder from the textile material: However, a rinse temperature that is too high, for example, above 43.33°C (110° F), tends to cause microcapsules to become unattached from a textile material.

Following the warm rinse, the rinse bath is drained from the treatment container. The treatment container is then filled with water having a temperature of about 26.67°C (80° F), to which a finishing agent, such as a lotion finish, is added. Finishing the microcapsule application process with a finish "coat" is preferred to "seal" the microcapsules in place on the textile material so as to avoid being easily washed from the textile material. The total cycle time for such a process for applying microcapsules to textile materials as in the present invention is approximately one hour. Accordingly, the present invention provides a method by which microcapsules are more thoroughly and evenly applied to a textile material in a cost-effective manner.

Textile materials formed by a process of the present invention include natural and synthetic textile fibers and yarns, woven and knitted fabrics, non-woven structures, and garments made from such fibers, yarns, and/or fabrics. Processes according to the present invention are particularly well suited for applying microcapsules to textile materials such as fine denier hosiery, including hosiery comprising nylon.

In an embodiment of the present invention, a process for applying microcapsules to a textile material comprises: placing the textile material in a treatment bath; contacting the textile material with the microcapsules; dispersing the microcapsules around and through the textile material with a dispersant; and adhering the dispersed microcapsules to the textile material with a binder, wherein the microcapsules are thoroughly dispersed and evenly applied to the textile material. Such a process for applying microcapsules to a textile material further comprises, prior to contacting the textile material with the microcapsules, measuring a predetermined weight of the microcapsules and diluting the predetermined weight of the microcapsules with warm water in a microcapsule-to-water ratio of approximately 10 to 1. In embodiments, the step of contacting the textile material with the microcapsules comprises physically dispersing the microcapsules around the textile material in the bath, such as by stirring the bath for three minutes.

In embodiments, a process for applying microcapsules to a textile material further comprises, after dispersing the microcapsules with the dispersant, heating the bath to a temperature in the range of about 26.67°C to 48.89°C (80° F to 120° F) for a period of between 8 and 20 minutes. In preferred embodiments, the step of heating the bath, after dispersing the microcapsules with the dispersant, comprises heating the bath to a temperature of 37.78°C (100° F) for approximately 8 minutes.

In embodiments, a process for applying microcapsules to a textile material further comprises, after adhering the dispersed microcapsules to the textile material with a binder, heating the bath to a temperature in the range of about 26.67°C to 48.89°C (80° F to 120° F) for a period of between 8 and 20 minutes. In preferred embodiments, the step of heating the bath, comprises heating the bath to a temperature of 37.78°C (100° F) for approximately 10 minutes.

In the present invention, after adhering the dispersed microcapsules to the textile material with a binder and heating the bath for a predetermined temperature and time, embodiments of a process for applying microcapsules to a textile material further comprise draining the treatment bath.

Embodiments of a process for applying microcapsules to a textile material further comprise rinsing the textile material. In embodiments, the step of rinsing the textile material further comprises rinsing the textile material with water having a temperature in the range of about 21.11°C to 43.33°C (70° F to 110° F) for a period of between 5 and 10 minutes. Preferably, the step of rinsing the textile material with water comprises rinsing the textile material with circulating water having a temperature of 26.67°C (80° F) for approximately 5 minutes.

In the present invention, after rinsing the textile material with water at a predetermined temperature and time, embodiments of a process for applying microcapsules to a textile material further comprise draining the treatment bath.

Embodiments of a process for applying microcapsules to a textile material further comprise substantially filling the treatment bath with water having a temperature of about 26.67°C (80° F). In embodiments, a process of the present invention further comprises adding a finishing agent to the treatment bath. In preferred embodiments, the finishing agent is a lotion finish.

In embodiments of the present invention, the microcapsules, the dispersant, and the binder each have an ionic charge, and the ionic charge of the microcapsules is opposite the ionic charge of the dispersant and the binder. In preferred embodiments, the microcapsules have an anionic charge and the dispersant and the binder each have a cationic charge.

In embodiments of the present invention, the microcapsules contain a moisturizing agent, a fragrance, or a combination of a moisturizing agent and a fragrance. In embodiments of the present invention, the microcapsules contain a vitamin or a mixture of different vitamins.

In embodiments of the present invention, the dispersant is silicone-based. In preferred embodiments, the silicone-based dispersant is a silicone finish.

In embodiments of the present invention, the binder is an acrylic.

In a embodiments of a process for applying microcapsules to a textile material of the present invention, prior to placing the textile material in a treatment bath, the textile material has completed a dyeing process.

In embodiments of the present invention, a process for applying microcapsules to a textile material comprises a finishing process for fine denier hosiery. In preferred embodiments, the fine denier hosiery comprises nylon.

In an embodiment of the present invention, a process for applying microcapsules to a textile material comprises: measuring a predetermined weight of the microcapsules and diluting the predetermined weight of the microcapsules with warm water in a microcapsule-to-water ratio of approximately 10 to 1; placing the textile material in a treatment bath; physically dispersing the microcapsules in the bath to contact the textile material with the microcapsules; dispersing the microcapsules around and through the textile material with a silicone-based dispersant; heating the bath to a temperature in the range of about 26.67°C to 48.89°C (80° F to 120° F) for a period of between 8 and 20 minutes; adding a binder to the bath to adhere the dispersed microcapsules to the textile material; heating the bath to a temperature in the range of about 26.67°C to 48.89°C (80° F to 120° F) for a period of between 8 and 20 minutes; draining the treatment bath; rinsing the textile material with water having a temperature in the range of about 21.11°C to 43.33°C (70° F to 110° F) for a period of between 5 and 10 minutes; draining the treatment bath; substantially filling the treatment bath with water having a temperature of about 26.67°C (80° F); and adding a finishing agent to the treatment bath, wherein the microcapsules, the dispersant, and the binder each have an ionic charge, and the ionic charge of the microcapsules is opposite the ionic charge of the dispersant and the binder, and wherein the microcapsules are thoroughly dispersed and evenly applied to the textile material.

In such an embodiment of the present invention, the microcapsules contain a moisturizing agent, a fragrance, or a combination of a moisturizing agent and a fragrance. In embodiments of the present invention, the microcapsules contain a vitamin or a mixture of different vitamins.

In such embodiments, a process for applying microcapsules to a textile material comprises a finishing process for fine denier hosiery.

In an embodiment of the present invention, a process for applying microcapsules to a textile material comprises: measuring a predetermined weight of the microcapsules and diluting the predetermined weight of the microcapsules with warm water in a microcapsule-to-water ratio of approximately 10 to 1; placing the textile material in a treatment bath; stirring the bath for three minutes to physically disperse the microcapsules and contact the textile material with the microcapsules; dispersing the microcapsules around and through the textile material with a dispersant, the dispersant being a silicone finish having a cationic charge; heating the bath to a temperature of 37.78°C (100° F) for approximately 8 minutes; adding an acrylic binder having a cationic charge to adhere the dispersed microcapsules to the textile material; heating the bath to a temperature of 37.78°C (100° F) for approximately 10 minutes; draining the treatment bath; rinsing the textile material with circulating water having a temperature of 26.67°C (80° F) for approximately 5 minutes; draining the treatment bath; substantially filling the treatment bath with water having a temperature of about 26.67°C (80° F); and adding a lotion finishing agent to the treatment bath, wherein the microcapsules are thoroughly dispersed and evenly applied to the textile material.

In such an embodiment of the present invention, the microcapsules contain a moisturizing agent, a fragrance, or a combination of a moisturizing agent and a fragrance. In embodiments of the present invention, the microcapsules contain a vitamin or a mixture of different vitamins.

In such embodiments, a process for applying microcapsules to a textile material comprises a finishing process for fine denier hosiery.

## Claims

1. A process for applying microcapsules to a textile material, comprising:
contacting the textile material with the microcapsules;
followed by dispersing the microcapsules around and through the textile material with a dispersant; and
followed by adhering the dispersed microcapsules to the textile material with a binder.

2. The process for applying microcapsules to a textile material of claim 1, further comprising, prior to contacting the textile material with the microcapsules, measuring a predetermined weight of the microcapsules and diluting the predetermined weight of the microcapsules with warm water in a microcapsule-to-water ratio of approximately 10 to 1.

3. The process for applying microcapsules to a textile material of claims 1 and 2, wherein contacting the textile material with the microcapsules comprises physically dispersing the microcapsules around the textile material in a treatment bath, preferably by stirring the bath for three minutes.

4. The process for applying microcapsules to a textile material of claims 1 to 3, further comprising, after dispersing the microcapsules with the dispersant in a treatment bath, heating the bath to a temperature in the range of about 26.7 to 48.9°C (80°F to 120° F) for a period of between 8 and 20 minutes, preferably to a temperature of 37.8°C (100°F) for approximately 8 minutes.

5. The process for applying microcapsules to a textile material of claims 1 to 4, further comprising, after adhering the dispersed microcapsules to the textile material with a binder in a treatment bath, heating the bath to a temperature in the range of about 26.7 to 48.9°C (80°F to 120°F) for a period of between 8 and 20 minutes, preferably to a temperature of 37.8°C (100°F) for approximately 10 minutes.

6. The process for applying microcapsules to a textile material of claims 1 to 5, further comprising, after adhering the dispersed microcapsules to the textile material with a binder, draining the treatment bath.

7. The process for applying microcapsules to a textile material of claims 1 to 6, further comprising, after draining the treatment bath, rinsing the textile material.

8. The process for applying microcapsules to a textile material of claim 7, wherein rinsing the textile material comprises rinsing the textile material with water having a temperature in the range of about 21.1°C to 43.3°C (70°F to 110°F) for a period of between 5 and 10 minutes and preferably comprises rinsing the textile material with circulating water having a temperature of 26.7°C (80°F) for approximately 5 minutes.

9. The process for applying microcapsules to a textile material of claims 1 to 8, further comprising draining the treatment bath after rinsing the textile material.

10. The process for applying microcapsules to a textile material of claims 1 to 9, further comprising, after draining the treatment bath, substantially filling the treatment bath with water having a temperature of about 26.7°C (80°F).

11. The process for applying microcapsules to a textile material of claims 1 to 10, further comprising, after substantially filling the treatment bath with water, adding a finishing agent to the treatment bath.

12. The process for applying microcapsules to a textile material of claim 11, wherein the finishing agent is a lotion finish.

13. The process for applying microcapsules to a textile material of claims 1 to 12, wherein the microcapsules, the dispersant, and the binder each have an ionic charge, and the ionic charge of the microcapsules is opposite to the ionic charge of the dispersant and the binder.

14. The process for applying microcapsules to a textile material of claim 13, wherein the microcapsules have an anionic charge and the dispersant and the binder each have a cationic charge.

15. The process for applying microcapsules to a textile material of claims 1 to 14, wherein the microcapsules contain a moisturizing agent or a fragrance or both

16. The process for applying microcapsules to a textile material of claims 1 to 15, wherein the microcapsules contain a vitamin or a mixture of different vitamins.

17. The process for applying microcapsules to a textile material of claims 1 to 16, wherein the dispersant is silicone-based.

18. The process for applying microcapsules to a textile material of claim 17, wherein the silicone-based dispersant is a silicone finish.

19. The process for applying microcapsules to a textile material of claims 1 to 18, wherein the binder is an acrylic.

20. The process for applying microcapsules to a textile material of claims 1 to 19, wherein prior to contacting the textile material with the microcapsules, the textile material has completed a dyeing process.

21. The process for applying microcapsules to a textile material of claims 1 to 20, wherein the process comprises a finishing process for fine denier hosiery.

22. The process for applying microcapsules to a textile material of claim 21, wherein the fine denier hosiery comprises nylon.

23. A process for applying microcapsules to a textile material, according to any preceding claim, comprising:
measuring a predetermined weight of the microcapsules and diluting the predetermined weight of the microcapsules with warm water in a microcapsule-to-water ratio of approximately 10 to 1;
placing the textile material in a treatment bath;
physically dispersing the microcapsules in the bath to contact the textile material with the microcapsules;
dispersing the microcapsules around and through the textile material with a silicone-based dispersant;
heating the bath to a temperature in the range of about 26.7 to 48.9°C (80°F to 120°F) for a period of between 8 and 20 minutes;
adding a binder to the bath to adhere the dispersed microcapsules to the textile material;
heating the bath to a temperature in the range of about 26.7 to 48.9°C (80°F to 120°F) for a period of between 8 and 20 minutes;
draining the treatment bath;
rinsing the textile material with water having a temperature in the range of about 21.1°C to 43.3 °C (70°F to 110°F) for a period of between 5 and 10 minutes;
draining the treatment bath;
substantially filling the treatment bath with water having a temperature of about 26.7°C (80°F); and
adding a finishing agent to the treatment bath,
wherein the microcapsules, the dispersant, and the binder each have an ionic charge, and the ionic charge of the microcapsules is opposite to the ionic charge of the dispersant and the binder, and
wherein the microcapsules are thoroughly dispersed and evenly applied to the textile material.

24. A process for applying microcapsules to a textile material, according to any preceding claim, the microcapsules having an anionic charge, comprising:
measuring a predetermined weight of the microcapsules and diluting the predetermined weight of the microcapsules with warm water in a microcapsule-to-water ratio of approximately 10 to 1;
placing the textile material in a treatment bath;
stirring the bath for three minutes to physically disperse the microcapsules and contact the textile material with the microcapsules;
dispersing the microcapsules around and through the textile material with a dispersant, the dispersant being a silicone finish having a cationic charge;
heating the bath to a temperature of 37.8°C (100°F) for approximately 8 minutes;
adding an acrylic binder having a cationic charge to adhere the dispersed microcapsules to the textile material;
heating the bath to a temperature of 37.8°C (100°F) for approximately 10 minutes;
draining the treatment bath;
rinsing the textile material with circulating water having a temperature of 26.7°C (80°F) for approximately 5 minutes;
draining the treatment bath;
substantially filling the treatment bath with water having a temperature of about 26.7°C (80°F); and
adding a lotion finishing agent to the treatment bath,
wherein the microcapsules are thoroughly dispersed and evenly applied to the textile material.

## Patentansprüche

1. Verfahren zum Auftragen von Mikrokapseln auf ein textiles Material, umfassend:
Kontaktieren des textilen Materials mit den Mikrokapseln; mit anschließendem Dispergieren der Mikrokapseln um das textile Material herum und durch dieses hindurch mit einem Dispersionsmittel; und anschließendes Anhaften der dispergierten Mikrokapseln an dem textilen Material mit einem Bindemittel.

2. Verfahren zum Auftragen von Mikrokapseln auf ein textiles Material nach Anspruch 1, weiterhin umfassend das Messen eines vorbestimmten Gewichtes der Mikrokapseln und Verdünnen des vorbestimmten Gewichtes der Mikrokapseln mit warmem Wasser in einem Mikrokapselzu-Wasser-Verhältnis von ungefähr 10 zu 1 vor dem Kontaktieren des textilen Materials mit den Mikrokapseln.

3. Verfahren zum Auftragen von Mikrokapseln auf ein textiles Material nach den Ansprüchen 1 und 2, worin das Kontaktieren des textilen Materials mit den Mirkokapseln das physikalische Dispergieren der Mikrokapseln um das textile Material herum in einem Behandlungsbad, bevorzugt durch Rühren des Bades für drei Minuten umfasst.

4. Verfahren zum Auftragen von Mikrokapseln auf ein textiles Material nach den Ansprüchen 1 bis 3, weiterhin umfassend das Erwärmen des Bades auf eine Temperatur in dem Bereich von etwa 26,7 bis 48,9°C (80°F bis 120°F) für eine Periode zwischen 8 und 20 min, bevorzugt auf eine Temperatur von 37,8°C (100°F) für ungefähr 8 min nach dem Dispergieren der Mikrokapseln mit dem Dispersionsmittel in einem Behandlungsbad.

5. Verfahren zum Auftragen von Mikrokapseln auf ein textiles Material nach den Ansprüchen 1 bis 4, weiterhin umfassend das Erwärmen des Bades auf eine Temperatur in dem Bereich von etwa 26,7 bis 48,9°C (80°F bis 120°F) für eine Periode zwischen 8 und 20 min, bevorzugt auf eine Temperatur von 37,8°C (100°F) für ungefähr 10 min, nach dem Anhaften der dispergierten Mikrokapseln an dem textilen Material mit einem Bindemittel.

6. Verfahren zum Auftragen von Mikrokapseln auf ein textiles Material nach den Ansprüchen 1 bis 5, weiterhin umfassend das Ablassen des Behandlungsbades nach dem Anhaften der dispergierten Mikrokapseln an dem textilen Material mit einem Bindemittel.

7. Verfahren zum Auftragen von Mikrokapseln auf ein textiles Material nach den Ansprüchen 1 bis 6, weiterhin umfassend das Spülen des textilen Materials nach dem Ablassen des Behandlungsbades.

8. Verfahren zum Auftragen von Mikrokapseln auf ein textiles Material nach Anspruch 7, worin das Spülen des textilen Materials das Spülen des textilen Materials mit Wasser mit einer Temperatur im Bereich von etwa 21,1 bis 43,3°C (70 bis 110°F) für eine Periode zwischen 5 und 10 min umfasst und bevorzugt das Spülen des textilen Materials mit zirkulierendem Wasser mit einer Temperatur von 26,7°C (80°C) für ungefähr 5 min umfasst.

9. Verfahren zum Auftragen von Mikrokapseln auf ein textiles Material nach den Ansprüchen 1 bis 8, weiterhin umfassend das Ablassen des Behandlungsbades nach dem Spülen des textilen Materials.

10. Verfahren zum Auftragen von Mikrokapseln auf ein textiles Material nach den Ansprüchen 1 bis 9, weiterhin umfassend im Wesentlichen Füllen des Behandlungsbades mit Wasser mit einer Temperatur von etwa 26,7°C (80°C) nach dem Ablassen des Behandlungsbades.

11. Verfahren zum Auftragen von Mikrokapseln auf ein textiles Material nach den Ansprüchen 1 bis 10, weiterhin umfassend die Zugabe eines Bearbeitungsmittels zum Behandlungsbad nach dem im Wesentlichen Füllen des Behandlungsbades mit Wasser.

12. Verfahren zum Auftragen von Mikrokapseln auf ein textiles Material nach Anspruch 11, worin das Bearbeitungsmittel ein Lotionsfinish ist.

13. Verfahren zum Auftragen von Mikrokapseln auf ein textiles Material nach den Ansprüchen 1 bis 12, worin die Mikrokapseln, das Dispersionsmittel und das Bindemittel jeweils eine ionische Ladung haben und die ionische Ladung der Mikrokapseln entgegengesetzt zu der ionischen Ladung des Dispersionsmittels und des Bindemittels ist.

14. Verfahren zum Auftragen von Mikrokapseln auf ein textiles Material nach Anspruch 13, worin die Mikrokapseln eine anionische Ladung haben und das Dispersionsmittel und das Bindemittel jeweils eine kationische Ladung haben.

15. Verfahren zum Auftragen von Mikrokapseln auf ein textiles Material nach den Ansprüchen 1 bis 14, worin die Mikrokapseln ein Feuchtigkeitsmittel oder einen Duftstoff oder beides enthalten.

16. Verfahren zum Auftragen von Mikrokapseln auf ein textiles Material nach den Ansprüchen 1 bis 15, worin die Mikrokapseln ein Vitamin oder eine Mischung von unterschiedlichen Vitaminen umfasst.

17. Verfahren zum Auftragen von Mikrokapseln auf ein textiles Material nach den Ansprüchen 1 bis 16, worin das Dispersionsmittel auf Silikonbasis ist.

18. Verfahren zum Auftragen von Mikrokapseln auf ein textiles Material nach Anspruch 17, worin das Dispersionsmittel auf Silikonbasis ein Silikonfinish ist.

19. Verfahren zum Auftragen von Mikrokapseln auf ein textiles Material nach den Ansprüchen 1 bis 18, worin das Bindemittel ein acrylisches ist.

20. Verfahren zum Auftragen von Mikrokapseln auf ein textiles Material nach den Ansprüchen 1 bis 19, worin vor dem Kontaktieren des textilen Materials mit den Mikrokapseln das textile Material ein Färbeverfahren vollendet hat.

21. Verfahren zum Auftragen von Mikrokapseln auf ein textiles Material nach den Ansprüchen 1 bis 20, worin das Verfahren ein Bearbeitungsverfahren für eine Maschenware mit feinem Denier umfasst.

22. Verfahren zum Auftragen von Mirkokapseln auf ein textiles Material nach Anspruch 21, worin die Maschenware mit feinem Denier Nylon umfasst.

23. Verfahren zum Auftragen von Mikrokapseln auf ein textiles Material nach einem der vorhergehenden Ansprüche, umfassend
Messen eines vorbestimmten Gewichtes der Mikrokapseln und Verdünnen des vorbestimmten Gewichtes der Mikrokapseln mit warmem Wasser in einem Mikrokapsel-zu-Wasser-Verhältnis von ungefähr 10 zu 1, Anordnen des textilen Materials in einem Behandlungsbad, physikalisches Dispergieren der Mikrokapseln im Bad zum Kontaktieren des textilen Materials mit den Mikrokapseln, Dispergieren der Mikrokapseln um das textile Material herum und durch dieses hindurch mit einem Dispersionsmittel auf Silikonbasis, Erwärmen des Bades auf eine Temperatur im Bereich von etwa 26,7 bis 48,9°C (80 bis 120°F) für eine Periode zwischen 8 und 20 min, Zugabe eines Bindemittels zum Bad zum Anhaften der dispergierten Mikrokapseln am textilen Material, Erwärmen des Bades auf eine Temperatur im Bereich von etwa 26,7 bis 48,9°C (80 bis 120°F) für eine Periode zwischen 8 und 20 min, Ablassen des Behandlungsbades, Spülen des textilen Materials mit Wasser mit einer Temperatur im Bereich von etwa 21,1 bis 43,3°C (70 bis 110°F) für eine Periode zwischen 5 und 10 min, Ablassen des Behandlungsbades, im Wesentlichen Füllen des Behandlungsbades mit Wasser mit einer Temperatur von etwa 26,7°C (80°F), und Zugabe eines Finishing-Mittels zum Behandlungsbad, worin die Mikrokapseln, das Dispersionsmittel und das Bindemittel jeweils eine ionische Ladung haben und die ionische Ladung der Mirkokapseln entgegengesetzt zur ionischen Ladung des Dispersionsmittels und des Bindemittels ist und worin die Mikrokapseln sorgfältig dispergiert und gleichmäßig auf das textile Material verteilt werden.

24. Verfahren zum Auftragen von Mikrokapseln auf ein textiles Material nach einem der vorhergehenden Ansprüche, wobei die Mikrokapseln eine anionische Ladung haben, umfassend
Messen eines vorbestimmten Gewichts der Mikrokapseln und Verdünnen des vorbestimmten Gewichtes der Mirkokapseln mit warmem Wasser in einem Mikrokapsel-zu-Wasser-Verhältnis von ungefähr 10 zu 1, Anordnen des textilen Materials in einem Behandlungsbad, Rühren des Bades für 3 min zum physikalischen Dispergieren der Mikrokapseln und Kontaktieren des textilen Materials mit den Mikrokapseln, Dispergieren der Mikrokapseln um das textile Material herum und durch dieses hindurch mit einem Dispersionsmittel, wobei das Dispersionsmittel ein Silikonfinish mit einer kationischen Ladung ist, Erwärmen des Bades auf eine Temperatur von 37,8°C (100°F) für ungefähr 8 min, Zugabe eines acrylischen Bindemittels mit einer kationischen Ladung zum Anhaften der dispergierten Mikrokapseln an dem textilen Material, Erwärmen des Bades auf eine Temperatur von 37,8°C (100°F) für ungefähr 10 min, Ablassen des Behandlungsbades, Spülen des textilen Materials mit zirkulierendem Wasser mit einer Temperatur von 26,7°C (80°F) für ungefähr 5 min, Ablassen des Behandlungsbades, im Wesentlichen Füllen des Behandlungsbädes mit Wasser mit einer Temperatur von etwa 26,7°C (80°F) und Zugabe eines Lotion-Finishing-Mittels zum Behandlungsbad, worin die Mikrokapseln sorgfältig dispergiert und gleichmäßig auf das textile Material aufgetragen werden.

## Revendications

1. Procédé pour appliquer des microcapsules à une matière textile, comprenant :
la mise en contact de la matière textile avec les microcapsules ;
suivie par la dispersion des microcapsules autour et à travers la matière textile avec un dispersant ; et
suivie par l'adhérence des microcapsules dispersées à la matière textile avec un liant.

2. Procédé pour appliquer des microcapsules à une matière textile selon la revendication 1, comprenant en outre, avant de mettre en contact la matière textile avec les microcapsules, la mesure d'un poids prédéterminé des microcapsules et la dilution du poids prédéterminé des microcapsules avec de l'eau chaude dans un rapport des microcapsules à l'eau d'approximativement 10 à 1.

3. Procédé pour appliquer des microcapsules à une matière textile selon les revendications 1 et 2, dans lequel la mise en contact de la matière textile avec les microcapsules comprend la dispersion physique des microcapsules autour de la matière textile dans un bain de traitement, de préférence en agitant le bain pendant trois minutes.

4. Procédé pour appliquer des microcapsules à une matière textile selon les revendications 1 à 3, comprenant en outre, après la dispersion des microcapsules avec le dispersant dans un bain de traitement, le chauffage du bain à une température dans la plage d'environ 26,7 à 48,9 °C (80 °F à 120 °F) pendant une période comprise entre 8 et 20 minutes, de préférence à une température de 37,8 °C (100 °F) pendant approximativement 8 minutes.

5. Procédé pour appliquer des microcapsules à une matière textile selon les revendications 1 à 4, comprenant en outre, après l'adhérence des microcapsules dispersées à la matière textile avec un liant dans un bain de traitement, le chauffage du bain à une température dans la plage d'environ 26,7 à 48,9°C (80 °F à 120 °F) pendant une période comprise entre 8 et 20 minutes, de préférence à une température de 37,8°C (100 °F) pendant approximativement 10 minutes.

6. Procédé pour appliquer des microcapsules à une matière textile selon les revendications 1 à 5, comprenant en outre, après l'adhérence des microcapsules à la matière textile avec un liant, la vidange du bain de traitement.

7. Procédé pour appliquer des microcapsules à une matière textile selon les revendications 1 à 6, comprenant en outre, après la vidange du bain de traitement, le rinçage de la matière textile.

8. Procédé pour appliquer des microcapsules à une matière textile selon la revendication 7, dans lequel le rinçage de la matière textile comprend le rinçage de la matière textile avec de l'eau ayant une température dans la plage d'environ 21,1 °C à 43,3 °C (70 °F à 110 °F) pendant une période comprise entre 5 et 10 minutes et de préférence comprend le rinçage de la matière textile avec de l'eau en circulation ayant une température de 26,7 °C (80 °F) pendant approximativement 5 minutes.

9. Procédé pour appliquer des microcapsules à une matière textile selon les revendications 1 à 8, comprenant en outre la vidange du bain de traitement après le rinçage de la matière textile.

10. Procédé pour appliquer des microcapsules à une matière textile selon les revendications 1 à 9, comprenant en outre, après la vidange du bain de traitement, le remplissage substantiel du bain de traitement avec de l'eau ayant une température d'environ 26,7 °C (80 °F).

11. Procédé pour appliquer des microcapsules à une matière textile selon les revendications 1 à 10, comprenant en outre, après le remplissage substantiel du bain de traitement avec de l'eau, l'addition d'un agent de finition au bain de traitement.

12. Procédé pour appliquer des microcapsules à une matière textile selon la revendication 11, dans lequel l'agent de finition est un apprêt en lotion.

13. Procédé pour appliquer des microcapsules à une matière textile selon les revendications 1 à 12, dans lequel les microcapsules, le dispersant et le liant ont chacun une charge ionique, et la charge ionique des microcapsules est opposée à la charge ionique du dispersant et du liant.

14. Procédé pour appliquer des microcapsules à une matière textile selon la revendication 13, dans lequel les microcapsules ont une charge anionique et le dispersant et le liant ont chacun une charge cationique.

15. Procédé pour appliquer des microcapsules à une matière textile selon les revendications 1 à 14, dans lequel les microcapsules contiennent un agent humidifiant ou un parfum ou les deux.

16. Procédé pour appliquer des microcapsules à une matière textile selon les revendications 1 à 15, dans lequel les microcapsules contiennent une vitamine ou un mélange de différentes vitamines.

17. Procédé pour appliquer des microcapsules à une matière textile selon les revendications 1 à 16, dans lequel le dispersant est à base de silicone.

18. Procédé pour appliquer des microcapsules à une matière textile selon la revendication 17, dans lequel le dispersant à base de silicone est un apprêt à base de silicone.

19. Procédé pour appliquer des microcapsules à une matière textile selon les revendications 1 à 18, dans lequel le liant est un acrylique.

20. Procédé pour appliquer des microcapsules à une matière textile selon les revendications 1 à 19, dans lequel avant la mise en contact de la matière textile avec les microcapsules, la matière textile a achevé un procédé de teinture.

21. Procédé pour appliquer des microcapsules a une matière textile selon les revendications 1 à 20, dans lequel le procédé comprend un procédé de finition pour des articles de bonneterie à deniers fins.

22. Procédé pour appliquer des microcapsules à une matière textile selon la revendication 21, dans lequel les articles de bonneterie à deniers fins comprennent du nylon.

23. Procédé pour appliquer des microcapsules à une matière textile, selon l'une quelconque des revendications précédentes, comprenant :
la mesure d'un poids prédéterminé des microcapsules et la dilution du poids prédéterminé des microcapsules avec de l'eau chaude dans un rapport des microcapsules à l'eau d'approximativement 10 à 1 ;
la mise en place de la matière textile dans un bain de traitement ;
la dispersion physique des microcapsules dans le bain de traitement pour mettre en contact la matière textile avec les microcapsules ;
la dispersion des microcapsules autour et à travers la matière textile avec un dispersant à base de silicone ;
le chauffage du bain à une température dans la plage d'environ 26,7 à 48,9 °C (80 °F à 120 °F) pendant une période comprise entre 8 et 20 minutes ;
l'addition d'un liant au bain pour faire adhérer les microcapsules dispersées à la matière textile ;
le chauffage du bain à une température dans la plage d'environ 26,7 à 48,9 °C (80 °F à 120 °F) pendant une période comprise entre 8 et 20 minutes ;
la vidange du bain de traitement ;
le rinçage de la matière textile avec de l'eau ayant une température dans la plage d'environ 21,1 °C à 43,3 °C (70 °F à 110 °F) pendant une période comprise entre 5 et 10 minutes ;
la vidange du bain de traitement ;
le remplissage substantiel du bain de traitement avec de l'eau ayant une température d'environ 26,7 °C (80 °F) ; et
l'addition d'un agent de finition au bain de traitement,
dans lequel les microcapsules, le dispersant et le liant ont chacun une charge ionique, et la charge ionique des microcapsules est opposée à la charge ionique du dispersant et du liant, et
dans lequel les microcapsules sont minutieusement dispersées et régulièrement appliquées sur la matière textile.

24. Procédé pour appliquer des microcapsules à une matière textile, selon l'une quelconque des revendications précédentes, les microcapsules ayant une charge anionique, comprenant :
la mesure d'un poids prédéterminé des microcapsules et la dilution du poids prédéterminé des microcapsules avec de l'eau chaude dans un rapport des microcapsules à l'eau d'approximativement 10 à 1 ;
la mise en place de la matière textile dans un bain de traitement ;
l'agitation du bain pendant trois minutes pour disperser physiquement les microcapsules et mettre en contact la matière textile avec les microcapsules ;
la dispersion des microcapsules autour et à travers la matière textile avec un dispersant, le dispersant étant un apprêt à base de silicone ayant une charge cationique ;
le chauffage du bain à une température de 37,8 °C (100 °F) pendant approximativement 8 minutes ;
l'addition d'un liant acrylique ayant une charge cationique pour faire adhérer les microcapsules dispersées à la matière textile ;
le chauffage du bain à une température de 37, 8 °C (100 °F) pendant approximativement 10 minutes ;
la vidange du bain de traitement ;
le rinçage de la matière textile avec de l'eau ayant une température de 26,7°C (80 °F) pendant approximativement 5 minutes ;
la vidange du bain de traitement ;
le remplissage substantiel du bain de traitement avec de l'eau ayant une température d'environ 26,7 °C (80 °F) ; et
l'addition d'un agent de finition en lotion au bain de traitement,
dans lequel les microcapsules sont minutieusement dispersées et régulièrement appliquées sur la matière textile.
